# EUROPEAN PATENT SPECIFICATION

(11) **EP 1 052 932 B1**
(45) Date of publication and mention of the grant of the patent: **03.12.2003**
(21) Application number: 97917364.8
(22) Date of filing: 24.04.1997
(51) Int. Cl.: A61B 17/34

(54) **MONITORING NEEDLE PUNCTURE IN SURGICAL OPERATIONS**
ÜBERWACHUNG DES EINSTICHS EINER KANÜLE BEI CHIRURGISCHEN OPERATIONEN
SURVEILLANCE DE LA PONCTION DANS UNE INTERVENTION CHIRURGICALE

(30) Priority: 25.04.1996 GR 96100139
(43) Date of publication of application: 22.11.2000
(73) Proprietor: Kontarinis, Dimitrios, 157 73 Athens (GR)
(72) Inventor: Kontarinis, Dimitrios, 157 73 Athens (GR)
(74) Representative: Georgiou-Kostakopoulos, Georgios
(86) International application number: GR9700014
(87) International publication number: WO97039680

(56) References cited:
- WO-A-95/02233
- DE-A- 2 922 239
- DE-A- 4 213 426
- US-A- 5 352 206
- US-A- 5 470 316

## Description

### BACKGROUND OF THE INVENTION

### 1. Field of the invention

The invention is a system that measures and relays to surgeons information about the puncture of tissues during surgical operations. A specific and immediate application of this invention is the objective diagnosis of the puncturing via a puncturing needle of the peritoneum and the entrance in the abdominal cavity at the beginning of a laparoscopic operation.

### 2. Description of the Related Art

Until now, at the beginning of a laparoscopic operation the surgeon punctures the tissues until he/she reaches the peritoneum so that he/she can insert the laparoscopic tools in the abdominal cavity. This puncture is done in two ways:
The first one is the 'open' method where the surgeon cuts open down to the peritoneum and punctures through using immediate visual feedback. This way is time consuming.
The second way is the 'blind' method during which the puncture is empirically diagnosed with the help of naturally generated vibrations that get relayed through the puncturing needle to the hand of the surgeon and excite his/her sense of touch.
The present invention not only measures naturally generated vibrations, but also processes and relays this information to the surgeon. In other words this system provides information that ensures the objective verification of puncturing that until now is done empirically. The importance of this system is even more obvious in the case of resurgery and operations on overweight patients. The invention is the realization of measurement of physical parameters of human manipulation during the puncture and manipulation of human tissue and other similar materials. The proposed system measures and uses these parameters in a similar way humans use them during bare-hand manipulation.
There has been considerable research in the field. For example, a system is described in DE Pat. No. 4213426 in which, the operation unit and the surgical tool are remotely connected and the pressure between the operation area and the surgical tool is measured.
U.S. Pat. No. 5,470,316 describes a system that provides an indication of whether the needle is in contact with the tissue and therefore the protective hollow shaft is positioned inside the puncturing needle. Furthermore, this system includes neither a sensor nor any other means for the conversion of pressure into an electric signal.
U.S. Pat. No. 5,352,206 refers to the detection of superatmospheric pressure only following the creation of a pneumoperitoneum. In addition, the measured parameter is pressure in the peritoneum.
DE Pat. No. 2922239 describes a system that uses a light emitting and detecting configuration to detect puncture.

In contrast to the existing inventions, the present invention is a system that measures the naturally produced vibrations during puncturing with a sensor coupled directly to the surgical tool that produces an electric signal and therefore allows for further processing. In addition, the present invention detects the puncture before the creation of a pneumoperitoneum

### DESCRIPTION OF THE INVENTION

The present invention offers information to the surgeon during the puncture with the puncturing needle. This puncture in order to be successful should go through the hypodermic fat, the transversalis fascia, the extraperitonial fat and the peritoneum. The process of puncture is a succession of punctures of hard tissues and penetrations through soft fatty tissues. At the instance of puncture the force that the needle exerts to the tissue, increases and decreases generating therefore fast transient vibrations. During the penetration through the fatty tissue the force remains almost constant. These vibrations generated by punctures are sensed by vibration sensors. It is evident by the description above that fast transient vibrations are also generated at contact and loss of contact with the tissue and that these vibrations are also sensed by vibration sensors.

### INDUSTRIAL APPLICATION OF THE INVENTION

A system of sensors capable of sensing vibrations can be placed on any location as long as it is coupled to the needle. This way the vibrations during puncture are measured. The signal produced contains the puncture information. This information is relayed, after it is processed to the user of the system via a display. The processing of the signal provides with the succession and timing sequence of the punctures of tissues and penetrations through fat and the exact position of the puncturing tool in the human body. More specifically with this system we observe in the initial puncture in laparoscopic surgery that the signal of penetration of fat differs from the signal of puncture of tissue and this is indicative of successful puncture. The similar mechanical interaction between the surgical tool and tissues of different patients generates vibrations in a restricted frequency spectrum. To exploit this, the system filters out the frequencies that are produced by the motion of the hand or the tool that are not related to puncture. The processed information is fed to a display that relays the desired information to the surgeon. The system also uses a database where puncturing data after each operation is stored. This database is also fed with data of mechanical models of human tissue. With this database the system provides with an extra indication for the surgeon through which he/she is informed of the probability of successful puncture. In other words the system compares the data acquired during the puncture to data stored in the database and decides from this comparison the probability of successful puncture. The use of a computer makes the system capable of transforming the sensor signal in various ways and presenting it to the surgeon through different displays: a monitor, a loud speaker or a haptic display that directly stimulates the sense of touch.

### BRIEF DESCRIPTION OF THE DRAWINGS

A preferred embodiment of the invention will be described with reference to the attached drawings:
Figure 1 shows events and phases during puncturing task along with a stylized diagram of the force exerted by the puncturing tool against the human tissue versus time.
Figure 2 shows the initial embodiment of the invention which includes a puncturing tool (1), a system of sensors (3) with insulating means (2), an amplifier (4) and an oscilloscope (5).
Figure 3 shows the addition of a band-pass filter (6) to the system.
Figure 4 shows the addition of a computer (8) with I/O capabilities (7), (9) to the system as well as the different presentations (10) to the user that are possible with the use of such a processing stage.

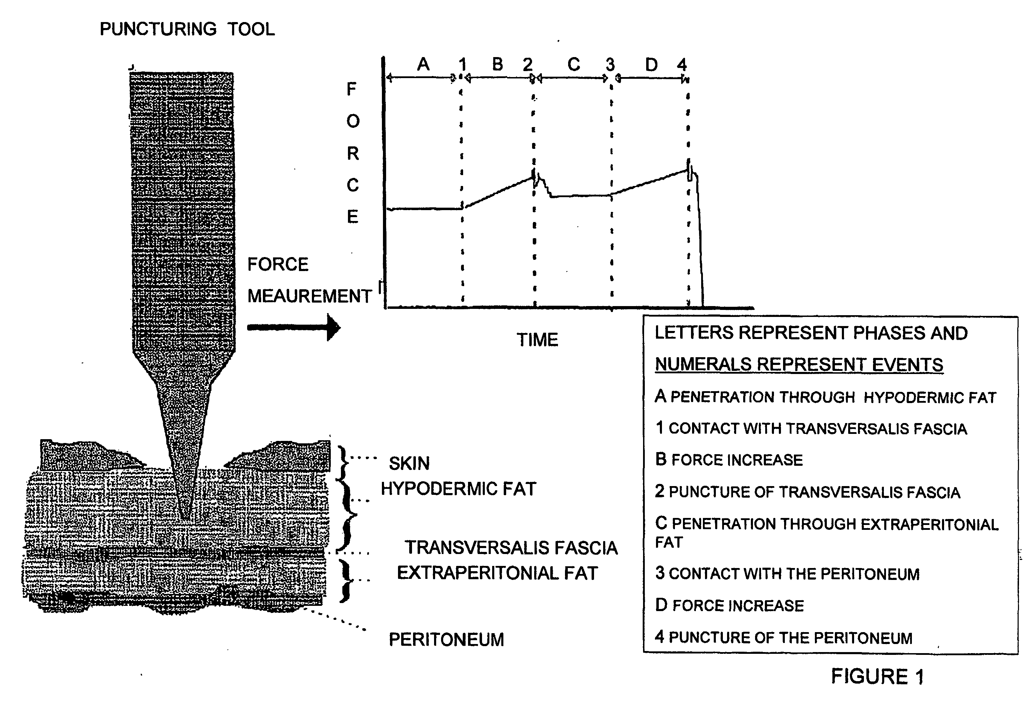

With the present invention we desire to offer information to the surgeon during the puncture with the puncturing needle. This puncture in order to be successful should go through the hypodermic fat, the transversalis fascia, the extraperitonial fat and the peritoneum The process of puncture includes the puncture of hard tissues and the penetration through soft fatty tissues. If we could measure the force on the puncturing needle we would see an increase in force until the puncture of tissue. At the instance of puncture this force would increase and decrease generating therefore fast transient vibrations. During the penetration through the fatty tissue the force would remain almost constant. The next stages would have similar progress as we can see in Figure 1.

### PREFERRED EMBODIMENTS OF THE INVENTION

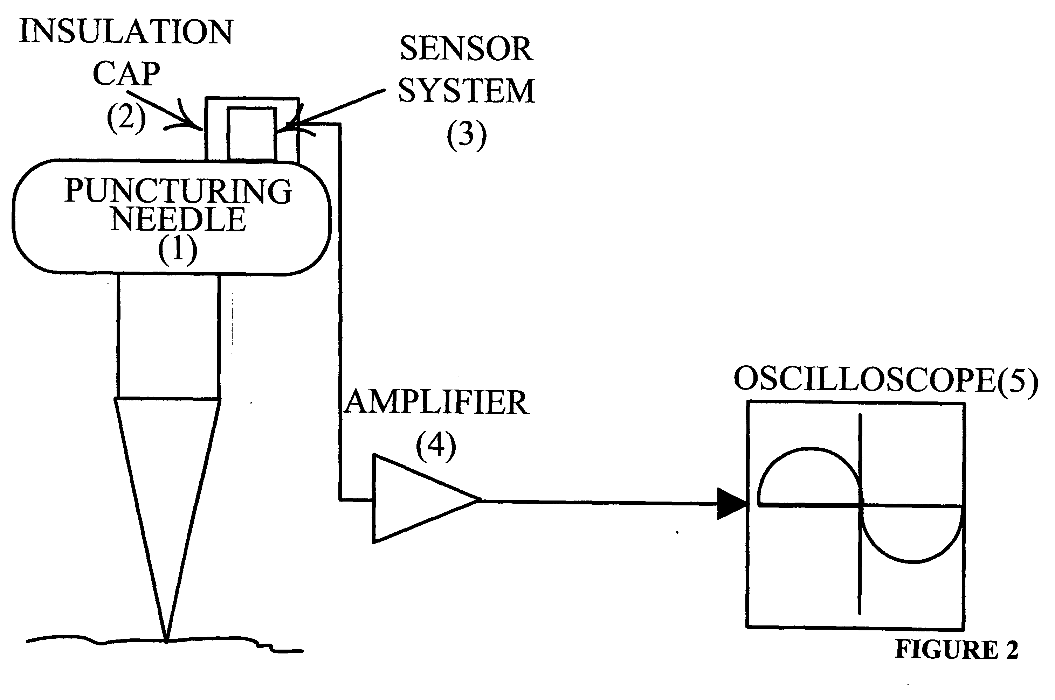

One preferred realisation of the invention is shown in Figure 2 and it includes initially a puncturing needle (1). A system of sensors (3) which in this embodiment comprises of a single sensor capable of sensing vibrations is placed on any location as long as it is coupled to the needle. In another embodiment a system of sensors for differential measurement of vibration as well as measurement of position displacement and velocity is used to further assist in the detection of puncture. This is done to differentiate vibrations produced by hand motion or tool motion to vibrations produced by puncture. There is also means (2) for insulating the sensor from external thermal and/or mechanical influence. This way the vibrations during puncture are measured. The signal produced by the sensor is amplified by an amplifier (4). In this embodiment the puncture information is relayed to the user of the system via a visual monitor such as an oscilloscope (5).

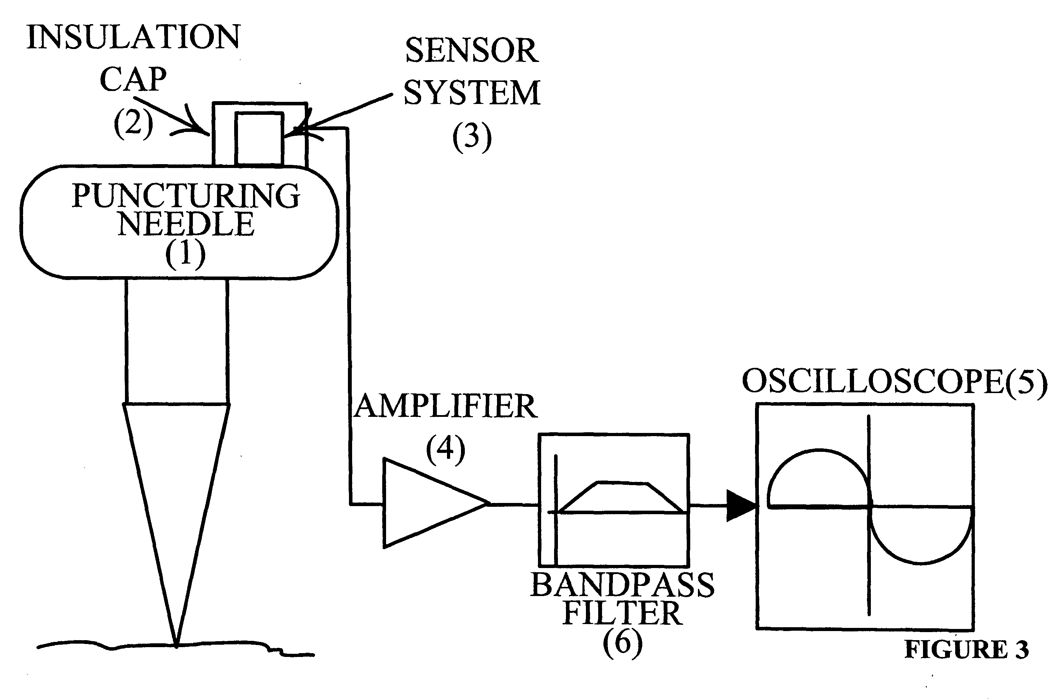

By exploiting the fact that the mechanical characteristics of human tissue do not differ by much, in other words the mechanical interaction between the surgical tool and tissues generates vibrations in a restricted frequency spectrum, we add as we can see in Figure 3 a band-pass filter (6) to filter out the frequencies that are not in this restricted spectrum. The diagnosis of the puncture is therefore much easier.

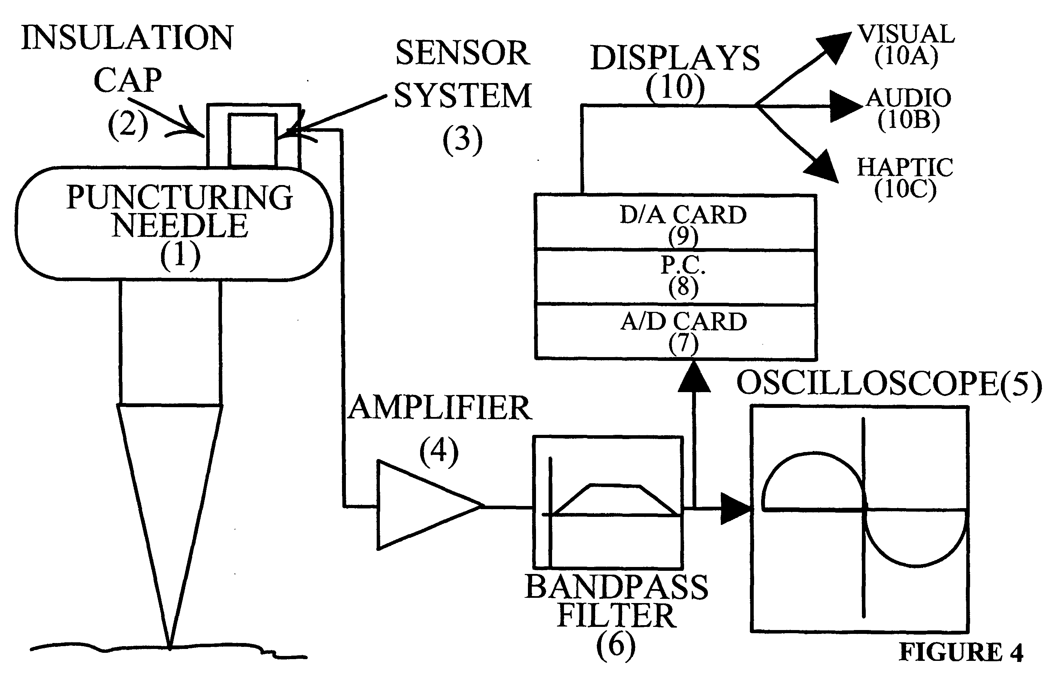

A further embodiment of the system shown in Figures 2 and 3 also includes a processing stage that contains a computer (8) with I/O capabilities such as an A/D (7) and a D/A (9) card that communicate with the sensor system as shown in Figure 4 The processed signal is fed to an interface that relays the desired information to the surgeon. With the use of a computer we can transform the sensor signal in various ways and present it to the surgeon through different displays. We are able to present it through a visual display (10a), an audio display (10b), or a haptic display (10c) that directly stimulates the sense of touch. All of these options and experimentations are possible with a processing stage such as a computer. It should be mentioned that those skilled in the art will envision modifications within the scope of this invention.

## Claims

1. System of monitoring needle puncture in surgical operations comprising of a system of sensors (3), coupled to any part of a puncturing tool (1), further comprising of a processing stage (6, 7, 8, 9) and a display system (10) of information **characterized in that** the system of sensors measures vibrations generated by the puncture and the processing stage uses said vibration signals to decide whether the puncture was successful.

2. System of monitoring needle puncture in surgical operations as claimed in claim 1 wherein the said system sensors' signal differs at the puncturing instance from the signal during the penetration in softer fat tissue.

3. System of monitoring needle puncture in surgical operations as claimed in claims 1 and 2 where in the said display system is visual (10A), audio (10B) and/or haptic (10C).

4. System of monitoring needle puncture in surgical operations as claimed in claims 1, 2 and 3 wherein the said system the sensor is coupled to any part of the puncturing tool in order to measure vibration during the attempted surgical puncturing of tissue and other similar material.

5. System of monitoring needle puncture in surgical operations as claimed in claims 1, 2 and 3 wherein a band-pass filter (6) is used so that the detection of puncturing is easier and safer.

6. System of monitoring needle puncture in surgical operations as claimed in claims 1, 2 and 3 where the processing stage includes I/O cards with communication capabilities with the sensor system so that the processed signal is relayed to the user's display to provide information about the puncture according to claim 3.

7. System of monitoring needle puncture in surgical operations as claimed in claims 1,2 and 3 wherein the system of sensors attaches to the puncturing tool and measures and relays information for the exact position and rate of penetration of the puncturing tool in the human body.

8. System of monitoring needle puncture in surgical operations as claimed in claims 1, 2 and 3 that comprises of the said system of sensors capable of detecting the contact or loss of contact with the punctured tissue.

## Patentansprüche

1. System zur Wahrnehmung von Nadeleinstichen bei chirurgischen Eingriffen, das das folgende umfasst, d.h. ein Sensorsystem (3), ahnkoppelbar an jeder Stelle des Einstichinstruments (1 ), eine Signalverarbeitungsstufe (6,7,8,9) und ein Indikationssystem für die erhältlichen Informationen (10), welches (als Gesamtsystem) dadurch gekenzeichnet wird, das es Vibrationen erfasst, die bei Nadeleinstichen entstehen, und die Signalverarbeitungsstufe nutzt diese Vibrationssignale um zu bestimmen ob der Einstich erfolgreich war.

2. System zur Wahrnehmung von Nadeleinstichen bei chirurgischen Eingriffen, das nach Pos. 1 dadurch charakterisiert wird, dass das von den Sensoren (während des Einstichs) ausgesandte Signal differenziert werden kann vom Signal, das während des Eindringens in den weicheren Fettschichten ausgesandt wird.

3. System zur Wahrnehmung von Nadeleinstichen bei chirurgischen Eingriffen, das nach Pos. 1 und 2 Informationen übertragen kann und diese optisch (10A), akustisch (10B) oder durch den Tastsinn (10C) indizieren kann.

4. System zur Wahrnehmung von Nadeleinstichen bei chirurgischen Eingriffen, das nach Pos. 1, 2 und 3 dadurch charakterisiert wird, dass es aus einem Sensor besteht angebracht an irgendeiner Stelle des Einstichinstruments mit dem Ziel Vibrationen zu erfassen, die während chirurgischer Einstiche ins Gewebe und ähnliche Materialien entstehen.

5. System zur Wahrnehmung von Nadeleinstichen bei chirurgischen Eingriffen, das nach Pos. 1,2 und 3, dadurch charakterisiert wird, dass es einen Signalunterdruckungsfilter (6) benutzt, damit die Verfolgung des Einstichs leichter und sicherer wird.

6. System zur Wahrnehmung von Nadeleinstichen bei chirurgischen Eingriffen, das nach Pos. 1, 2 und 3, dadurch charakterisiert wird, dass es in der Signalverarbeitungsphase PC-Karten (I/O) hinzugefügt werden mit Kommunikationsmöglichkeiten mit dem Sensorensystem, damit das verarbeitete Signal in den Monitor des Nutzers übertragen wird und ihm Informationen bezüglich des Einstichs nach Pos. 3 vermittelt.

7. System zur Wahrnehmung von Nadeleinstichen bei chirurgischen Eingriffen gemäss der Pos. 1,2 und 3, wobei das Sensorensystem ans Einstichinstrument angebracht wird und Informationen zur genauen Position und zum Durchdringungstempo des Einstichinstruments in den menschlichen Körper registriert und vermittelt.

8. System zur Wahrnehmung von Nadeleinstichen bei chirurgischen Eingriffen, das nach Pos 1,2, und 3, dadurch charakterisiert wird, dass es aus einem Sensorensystem besteht, das den Kontakt oder das Ende des Kontaktes mit dem durchstochenen Gewebe registriert.

## Revendications

1. Système de surveillance d'une perforation avec une aiguille, s'appliquant à des opérations chirurgicales, et qui contient un système de senseurs, uni à n'importe quel point de l'instrument de perforation, un stade d'élaboration du signal et un système qui indique des informations extraites, lequel (système dans son ensemble) se **caractérise par le fait qu'**il mesure des vibrations qui se produisent par la perforation, et le stade d'élaboration utilise ces signaux de vibration pour préciser si la perforation a réussi.

2. Système de surveillance d'une perforation avec une aiguille, s'appliquant à des opérations chirurgicales, lequel, selon la revendication 1, se **caractérise par le fait que**, le signal émis par le système de senseurs au moment de la perforation, se différencie du signal émis pendant la pénétration dans les couches molles de graisse.

3. Système de surveillance d'une perforation avec une aiguille, s'appliquant à des opérations chirurgicales, qui, selon les revendications 1 et 2, transfert les informations à travers des indications visuelles, auditives et tactiles.

4. Système de surveillance d'une perforation avec une aiguille, s'appliquant à des opérations chirurgicales, qui se **caractérise par le fait qu'**il est constitué par un senseur qui est joint sur n'importe quel point de l'instrument afin de mesurer des vibrations pendant l'entreprise perforation chirurgicale du tissu et des autres matériaux connexes.

5. Système de surveillance d'une perforation avec une aiguille, s'appliquant à des opérations chirurgicales, qui se **caractérise par le fait qu'**on utilise un filtre d'excision pour que le dépistage de la perforation soit plus facile et plus assuré.

6. Système de surveillance d'une perforation avec une aiguille, s'appliquant à des opérations chirurgicales, qui se **caractérise par le fait que**, pendant le stade d'élaboration, on ajoute des cartes d'entrée et de sortie (1/0) qui ont des possibilités de communication avec le système des senseurs, de sorte que le signal élaboré soit transféré sur l'écran de l'usager et qu'il procure des informations concernant la perforation, selon la revendication 3.

7. Système de surveillance d'une perforation avec une aiguille, s'appliquant à des opérations chirurgicales, qui se **caractérise par le fait que** le système des senseurs est ajusté sur l'instrument chirurgical et il enregistre et transmet des informations, concernant la position exacte et le rythme de perforation de l'instrument de perforation dans le corps humain.

8. Système de surveillance d'une perforation avec une aiguille, s'appliquant à des opérations chirurgicales, selon les revendications 1, 2 et 3, lequel se **caractérise par le fait qu'**il est constitué d'un système des senseurs, qui dépiste le contact ou la fin du contact avec le tissu qui est en train d'être perforé.
